# EUROPEAN PATENT SPECIFICATION

(11) **EP 3 643 325 B1**
(45) Date of publication and mention of the grant of the patent: **10.04.2024**
(21) Application number: 19209609.7
(22) Date of filing: 06.10.2017
(51) Int. Cl.: A61K 47/10, A61K 47/26, A61K 47/32, A61K 47/36, A61K 9/10, A61K 47/38, A61K 9/16, A61K 31/4178

(54) **A COMPOSITION COMPRISING FURAZIDIN AND A METHOD OF ITS MANUFACTURING**
ZUSAMMENSETZUNG ENTHALTEND FURAZIDIN SOWIE DEREN HERSTELLUNGSMETHODE
COMPOSITION COMPRENANT DE LA FURAZIDINE ET SON PROCÉDÉ DE FABRICATION

(30) Priority: 10.10.2016 PL 41904716; 08.05.2017 PL 42150517
(43) Date of publication of application: 29.04.2020
(62) Divisional of application: 17797439.1
(73) Proprietor: Przedsiebiorstwo Produkcji Farmaceutycznej Hasco-Lek S.A., 51-131 Wroclaw (PL); Centrum Badawczo-Rozwojowe Novasome sp. z o.o., 51-423 Wroclaw (PL)
(72) Inventor: Paduszynski, Piotr, 98-346 Skomlin (PL); Czescik, Katarzyna, 54-104 Wroclaw (PL); Potaczek, Piotr, 54-129 Wroclaw (PL); Han-Marek, Malgorzata, 02-999 Warszawa (PL); Han, Tomasz, 55-095 Szczodrze (PL); Han, Stanislaw, 51-423 Wroclaw (PL)
(74) Representative: Krekora, Magdalena

(56) References cited:
- RU-A- 2015 132 729
- US-A- 4 500 516
- "Lietosanas instrukcija / Furamag - instructions for use", Zalu valst agentura / Latvian Medical State Agency , 23 January 2015 (2015-01-23), XP002779595, Retrieved from the Internet: URL:https://www.zva.gov.lv/zalu-registrs/? iss=1&lang=lv&q=furamag&ON=&SN=&NAC=on&RN= &ESC=on&AK=&SAT=on&RA=&DEC=on&LB=&PIM=on&M FR=&MDO=&IK= [retrieved on 2018-03-27]
- DATABASE CAPLUS [Online] CHEMICAL ABSTRACTS SERVICE, COLUMBUS, OHIO, US; GROSHOVYI, T. A. ET AL: "Optimization of tablet production technology IX. Use of a symmetric composition design for studying quantitative factors in manufacture of enterosoluble furagin tablets", XP002779596, retrieved from STN Database accession no. 1987:9323 & GROSHOVYI, T. A. ET AL: "Optimization of tablet production technology IX. Use of a symmetric composition design for studying quantitative factors in manufacture of enterosoluble furagin tablets", FARMATSEVTICHNII ZHURNAL (KIEV) , (5), 50-4 CODEN: FRZKAP; ISSN: 0367-3057, 1986,

## Description

### TECHNICAL FIELD

The object of the present invention is a pharmaceutical oral composition comprising furazidin, and a method of manufacturing it.

### BACKGROUND ART

Furazidin (Furaginum) is a derivative of nitrofuran, broad-spectrum chemotherapeutic agent. It acts against both Gram-positive and Gram-negative bacteria. It demonstrates bacteriostatic effect inter alia on *Staphylococcus aureus, Staphylococcus epidermidis, Staphylococcus faecalis, Salmonella, Shigella, Proteus, Klebsiella, Escherichia, Enterobacter.* Furazidin is used for treatment and prophylaxis of acute and chronic urinary tract infections.

Furazidin is administered in oral form - in the form of tablets. Tablets comprise corn starch, sucrose, colloidal silicon dioxide and stearic acid as excipients. The dosage form of a tablet is not very suitable for some groups of patients who have problems with swallowing e.g. children or elderly persons. Furthermore, US4500516A discloses capsules comprising furazidin granules.

It has been found unexpectedly, that it is possible to deliver to those groups of patients a new oral dosage form which will be more suitable for them.

### DISCLOSURE OF THE INVENTION

Oral pharmaceutical composition comprising furazidin according to the invention is in the form of powder or/and granules or/and coated granules, and it comprises from 0.5% by weight to 95% by weight of furazidin and it comprises at least one bulking agent in an amount from 5% by weight to 99% by weight chosen from the group consisting of starch, gelatinized starch, microcrystalline cellulose, lactose, glucose, mannitol, sorbitol, talc, anhydrous colloidal silica, dextrins or/and their mixture.

The composition according to the invention may be directly administered to a patient. It may be used for preparation of oral suspension or/and a filling of hard capsule. It may be used for preparation of pellets or/and coated pellets or/and minitablets or/and coated minitablets, and they may constitute a filling of hard capsules. The composition according to the invention may be suspended in a dispersion phase what enables filling of soft capsule or/and hard capsule.

The composition has non-modified release characteristics.

The composition has modified release characteristics. The term "modified release" shall be understood as prolonged release, delayed release, pulsatile release or accelerated release.

The composition comprises at least one compound having binding or/and coating properties in an amount from 0.1% by weight to 30 % by weight chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides. Saccharides used in the composition are e.g. sucrose or/and dextrins, polyhydric alcohols are e.g. sorbitol, mannitol, and chemically modified cellulose derivatives are e.g. hydroxypropyl cellulose, hydroxypropyl methylcellulose, cellulose phthalates or/and cellulose acetate. Polyvinylpyrrolidones used in the compositions are e.g. povidone K15/17 or/and povidone K25, whereas polyethylene oxides are e.g. PEG 400 or/and PEG 6000.

The composition comprises at least one compound having emulsifying properties in an amount from 0.1% by weight to 45% by weight chosen from the group consisting of phospholipids, polyoxyethylene sorbitan derivatives, fatty acids and/or fatty alcohols. Phospholipids used in the compositions are soya lecithin or/and sunflower lecithin, and sorbitan derivatives are e.g. sorbitan sesquioleate. Fatty acids used in the composition are e.g. oleic acid, fatty alcohols are e.g. oleic alcohol.

The composition comprises at least one compound chosen from the group of surfactants (e.g. sodium lauryl sulfate) in an amount from 0.1% by weight to 15% by weight.

The composition comprises at least one lubricant in an amount from 0.1% by weight to 10% by weight. Lubricants used are e.g. stearic acid, magnesium stearate, talc or/and their mixture.

The composition comprises at least one substance chosen from the group consisting of sweetening agents, flavoring agents and buffering agents in an amount from 0.01% by weight to 25% by weight. The composition may comprise just one of these agents or any of their mixtures. Sweetening agents used are e.g. sucrose, acesulfame K, sodium saccharin or/and sucralose. Flavoring agents comprised in the composition are e.g. orange, cherry, lemon or/and banana flavor, and buffering agents are e.g. sodium dihydrogen phosphate, sodium hydrogen phosphate, citric acid or/and sodium citrate.

The object of the present invention is also a method of manufacturing oral composition comprising furazidin and bulking agent as defined above which consists in that a water or/and organic solution of the substance having binding properties chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides having concentration of 0.1% by weight to 30 % by weight is prepared, then furazidin in an amount from 0.5% by weight to 95 % by weight is mixed with the substance having emulsifying properties and/or with the bulking agent, then the mixture of furazidin with the substance having emulsifying properties and/or with the bulking agent is granulated with addition of the binding substance solution, after granulation the obtained granules are calibrated and dried, and then they are mixed with bulking agent and/or a lubricant and/or a surfactant and/or a sweetening agent and/or a flavoring agent and/or a buffering agent. The composition obtained by this method is in the form of granules.

Granules directly after drying or after drying and calibration, and before mixing with other substances is coated with coating agents.

The object of the present invention is also a method of manufacturing the composition comprising furazidin and bulking agent as defined above in the form of a powder. The method consists in that furazidin in an amount from 0.5 to 95% by weight is mixed with a bulking agent chosen from the group consisting of starch, gelatinized starch, microcrystalline cellulose, lactose, glucose, mannitol, sorbitol, talc, anhydrous colloidal silica, dextrins or/and their mixture or/and additionally with a binding agent chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides or/and an emulsifying agent chosen from the group consisting of phospholipids, polyoxyethylene sorbitan derivatives, fatty acids and/or fatty alcohols or/and a surfactant or/and a lubricant or/and a sweetening agent or/and a flavoring agent or/and a buffering agent.

The examples below illustrate the invention.

### Example 1

Quantitative-qualitative composition of the formulation is depicted below.

| **No.** | **Name of the ingredient** | **% wt/wt** |
|---|---|---|
| 1. | Furazidin | 50 |
| 2. | Gelatinized corn starch | 30.5 |
| 3. | Sucrose | 16.9 |
| 4. | Anhydrous colloidal silica | 0.5 |
| 5 | Stearic acid 50 | 2.1 |
| | SUM: | 100 |

Description of technological process: in the apparatus for wet granulation e.g. in a mixer granulator the ingredients 1 and 2 were placed. A solution of binding agent being a water solution of the ingredient 3 was prepared. The ingredients 1 and 2 were mixed until smooth, however not shorter than 5 minutes. Granulation was performed with use of the water solution of the ingredient 3. The water solution of the ingredient 3 shall have the concentration from 50 to 100 % wt/wt, and it is possible to use maximal ratio of the solvent : ingredient 3 as 1:2. After granulation the granules obtained were calibrated and dried to achieve dryness from 0.5 to 15%. In case of need the dried granules were calibrated. The ingredient 4 was added. The whole was mixed until smooth, but not less than 3 min. The ingredient 5 was added. The whole was mixed until smooth, but not less than 3 min. The product obtained may be used as a filling for hard capsules or may be used for obtaining pellets or minitablets, where may then constitute a filling for hard capsules.

### Example 2

Quantitative-qualitative composition of the formulation is depicted below.

| **No.** | **Name of the ingredient** | **% wt/wt** |
|---|---|---|
| 1. | Furazidin | 1,111 |
| 2. | Gelatinized corn starch | 0,678 |
| 3. | Sucrose | 0,376 |
| 4. | Anhydrous colloidal silica | 0,011 |
| 5. | Stearic acid 50 | 0,047 |
| 6. | Orange flavor | 2,502 |
| 7. | Acesulfame potassium | 0,084 |
| 8. | Sodium saccharin | 0,084 |
| 9. | Sucralose | 0,133 |
| 10. | Riboflavin | 0,018 |
| 11. | Sorbitol (powder) | 94,956 |
| | SUM: | 100 |

Description of technological process: in the apparatus for wet granulation e.g. in a mixer granulator the ingredients 1 and 2 were placed. A solution of binding agent being a water solution of the ingredient 3 was prepared. The ingredients 1 and 2 were mixed until smooth, however not shorter than 5 minutes. Granulation was performed with use of the water solution of the ingredient 3. The water solution of the ingredient 3 shall have the concentration from 50 to 200 % wt/wt, and it is possible to use maximal ratio of the solvent : ingredient 3 as 1:2. After granulation the granules obtained were calibrated and dried to achieve dryness from 0.5 to 15%. In case of need the dried granules were calibrated. The ingredient 4 was added. The whole was mixed until smooth, but not less than 3 min. The ingredients 7 to 11 were added and mixed until smooth, but but not less than 3 min. It is possible to mix each ingredient in a separate cycle of mixing or/and all ingredients together. The ingredient 5 was added. The whole was mixed until smooth, but not less than 3 min. The mass obtained was filled into sachets. The product obtained may be administered directly to a patient or/and used for preparation of a suspension directly before administration.

### Example 3

Quantitative-qualitative composition of the formulation is depicted below.

| **No.** | **Name of the ingredient** | **% wt/wt** |
|---|---|---|
| 1. | Furazidin | 2,77 |
| 2. | Lactose monohydrate | 1,94 |
| 3. | Polyvinylpyrrolidone | 1,11 |
| 4. | Talc | 0,03 |
| 5. | Magnesium stearate | 0,33 |
| 6. | Orange flavor | 3,12 |
| 7. | Sucrose | 55,38 |
| 8. | Sorbitol (powder) | 35,33 |
| | SUM: | 100 |

Description of technological process: in the apparatus for wet granulation e.g. in a mixer granulator the ingredients 1 and 2 were placed. A solution of binding agent being a water solution of the ingredient 3 was prepared. The ingredients 1 and 2 were mixed until smooth, however not shorter than 5 minutes. Granulation was performed with use of the water solution of the ingredient 3. The water solution of the ingredient 3 shall have the concentration from 5 to 50 % wt/wt. After granulation the granules obtained were calibrated and dried to achieve dryness from 0.5 to 15%. In case of need the dried granules were calibrated. The ingredient 4 was added. The whole was mixed until smooth, but not less than 3 min. The ingredients 6 to 8 were added and mixed until smooth, but but not less than 3 min. It is possible to mix each ingredient in a separate cycle of mixing or/and all ingredients together. The ingredient 4 and 5 was added. The whole was mixed until smooth, but not less than 3 min. The mass obtained was filled into sachets. The product obtained may be administered directly to a patient or/and used for preparation of a suspension directly before administration.

### Example 4

| **No.** | **Name of the ingredient** | **% wt/wt** |
|---|---|---|
| 1. | Furazidin | 2,5 |
| 2. | Lactose monohydrate | 1,75 |
| 3. | Polyvinylpyrrolidone | 1 |
| 4. | Talc | 0,025 |
| 5. | Magnesium stearate | 0,3 |
| 6. | Orange flavor | 5,6 |
| 7. | Sucrose | 61,1 |
| 8. | Mannitol | 25 |
| 9. | Citric acid | 1 |
| 10. | Sodium citrate | 1,35 |
| 11. | Mixture of polyoxyethylene derivatives of sorbitan and oleic acid | 0,375 |
| | SUM: | 100 |

Description of technological process: A water solution of the ingredients 3 and 11 was prepared. In the apparatus for wet granulation the ingredients 1, 2 and 7 were placed. The whole was mixed until smooth, however not shorter than 3 minutes. It is possible to mix all ingredients together or separately. The mixture was granulated with use of the water solution of the ingredient 3 and 11. In case of need the obtained granules were calibrated. The granules were dried to achieve dryness from 0.5 to 25%. In case of need granules were calibrated. The ingredients 6, 8, 9 and 10 were added to the obtained granules. The whole was mixed until smooth, but not less than 3 min. It is possible to mix each ingredient in a separate cycle of mixing or/and all ingredients together. The ingredient 4 and 5 was added. The whole was mixed until smooth, but not less than 3 min. The mass obtained was filled into sachets. The product obtained may be administered directly to a patient or/and used for preparation of a suspension directly before administration.

### Example 5

| **No.** | **Name of the ingredient** | **% wt/wt** |
|---|---|---|
| 1. | Furazidin | 13,692 |
| 2. | Lactose monohydrate | 0,479 |
| 3. | Mixture of copolymers of methacrylic or/and acrylic acid | 0,685 |
| 4. | Talc | 0,021 |
| 5. | Magnesium stearate | 0,014 |
| 6. | Orange flavor | 1,534 |
| 7. | Sucrose | 41,076 |
| 8. | Sorbitol | 41,076 |
| 9. | Citric acid | 0,548 |
| 10. | Sodium citrate | 0,739 |
| 11. | Mixture of polyoxyethylene derivatives of sorbitan and oleic acid | 0,137 |
| | SUM: | 100 |

Description of technological process: A dispersion of the ingredients 3 and 11 was prepared. In the apparatus for wet granulation the ingredients 1, 2 and part of portion of the ingredient 7 were placed. It is possible to use the ingredient 7 in proportion from 0 to 100%. Granulation was performed with use of the solution of the ingredients 3 and 11. Obtained granules were calibrated in case of need, and then dried to achieve dryness from 0.5 to 25%. After drying the granules obtained were calibrated in case of need. The rest of the portion of the ingredient 7 was added to the obtained granules. (if 100% of the portion of the ingredient 7 is used for granulation, this step shall be omitted). Next ingredients 6, 9 and 10 were added to the mixture. The whole was mixed until smooth, but not less than 3 min. It is possible to mix each ingredient in a separate cycle of mixing or/and all ingredients together. The ingredient 4 and 5 was added. The whole was mixed until smooth, but not less than 3 min. It is possible to mix each ingredient in a separate cycle of mixing or/and all ingredients together. The product obtained may be administered directly to a patient or/and used for preparation of a suspension directly before administration.

### Example 6

| **No.** | **Name of the ingredient** | **% wt/wt** |
|---|---|---|
| 1. | Furazidin | 13,692 |
| 2. | Lactose monohydrate | 0,479 |
| 3. | Mixture of copolymers of methacrylic or/and acrylic acid | 0,685 |
| 4. | Talc | 0,021 |
| 5. | Magnesium stearate | 0,014 |
| 6. | Orange flavor | 1,534 |
| 7. | Sucrose | 41,076 |
| 8. | Sorbitol | 41,076 |
| 9. | Citric acid | 0,548 |
| 10. | Sodium citrate | 0,739 |
| 11. | Mixture of polyoxyethylene derivatives of sorbitan and oleic acid | 0,137 |
| | SUM: | 100 |
| | | |

Description of technological process : A water dispersion of the ingredient 7 was prepared. The water solution of the ingredient 7 shall have the concentration from 50 to 200 % wt/wt, and it is possible to use maximal ratio of the solvent : ingredient 3 as 1:2. Non-used part of the portion of the ingredient 7 will be used in the next step of the process. In the apparatus for wet granulation the ingredients 1 and 2 were placed. It is possible to use the ingredient 8 in proportion from 0 to 100%. Granulation was performed with use of the solution of the ingredient 7. Obtained granules were calibrated in case of need. They were dried to achieve dryness from 0.5 to 25%. A water solution of the ingredient 3 was prepared. Dried granules were fluid coated with the solution of the ingredient 3. To the obtained granules the remaining portions of the ingredients 7 and 8 were added, and then 8 and 9. The whole was mixed until smooth, but not less than 3 min. It is possible to mix each ingredient in a separate cycle of mixing or/and all ingredients together. The ingredients 4 and 5 were added. It is possible to mix each ingredient in a separate cycle of mixing or/and all ingredients together. The product obtained may be administered directly to a patient or/and used for preparation of a suspension directly before administration.

### Example 7

| **No.** | **Name of the ingredient** | **% wt/wt** |
|---|---|---|
| 1. | Furazidin | 64,412 |
| 2. | Sucrose | 32,206 |
| 3. | Mixture of copolymers of methacrylic or/and acrylic acid | 3,221 |
| 4. | Talc | 0,097 |
| 5. | Magnesium stearate | 0,064 |
| | SUM: | 100 |

Description of technological process: A water solution of the ingredient 3 was prepared. In the apparatus for wet granulation the ingredients 1 and 2 were placed. The ingredients 1 and 2 were mixed. Granulation was performed in a fluid bed with use of the solution of the ingredient 3. In case of need the obtained granules were calibrated. The ingredients 4 and 5 were added to the obtained granules. The whole was mixed until smooth, but not less than 3 min. It is possible to mix each ingredient in a separate cycle of mixing or/and all ingredients together. The ingredient 4 and 5 was added. The whole was mixed until smooth, but not less than 3 min. The product obtained is intended for obtaining pellets or minitablets, that may be coated and constitute a filling for hard capsules or/and may be suspended in a dispersion phase what enables filling of soft capsule or/and hard capsule.

## Claims

1. A pharmaceutical oral composition comprising furazidin, **characterized in that** it is in the form of powder or/and granules or/and coated granules, and it comprises from 0.5% by weight to 95% by weight of furazidin and it comprises at least one bulking agent in an amount from 5% by weight to 99% by weight chosen from the group consisting of starch, gelatinized starch, microcrystalline cellulose, lactose, glucose, mannitol, sorbitol, anhydrous colloidal silica, talc, dextrins or/and their mixture, and it comprises at least one compound having binding or/and coating properties chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides in an amount from 0.1% by weight to 30 % by weight.

2. The composition according to claim 1, **characterized in that** it has non-modified release characteristics.

3. The composition according to claim 1, **characterized in that** it has modified release characteristics.

4. The composition according to any of the preceding claims, **characterized in that** it comprises at least one compound having emulsifying properties in an amount from 0.1% by weight to 45% by weight chosen from the group consisting of phospholipids, polyoxyethylene sorbitan derivatives, fatty acids and/or fatty alcohols.

5. The composition according to any of the preceding claims, **characterized in that** it comprises at least one compound chosen from the group of surfactants in an amount from 0.1% by weight to 15% by weight.

6. The composition according to any of the preceding claims, **characterized in that** it comprises at least one lubricant in an amount from 0.1% by weight to 10% by weight.

7. The composition according to any of the preceding claims, **characterized in that** it comprises at least one substance chosen from the group consisting of sweetening agents , flavoring agents and buffering agents in an amount from 0.01% by weight to 25% by weight.

8. A method of manufacturing the composition as defined in the claims 1-7 comprising furazidin and bulking agent, **characterized in that** a water or/and organic solution of the substance having binding properties chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides having concentration of 0.1% by weight to 30 % by weight is prepared, then furazidin in an amount from 0.5% by weight to 95 % by weight is mixed with the substance having emulsifying properties and/or with the bulking agent, then the mixture of furazidin with the substance having emulsifying properties and/or with the bulking agent is granulated with addition of the binding substance solution, after granulation the obtained granules are calibrated and dried, and then they are mixed with bulking agent and/or a lubricant and/or a surfactant and/or a sweetening agent and/or a flavoring agent and/or a buffering agent.

9. The method according to claim 8, **characterized in that** granules directly after drying or after drying and calibration, and before mixing with other substances are coated with coating agents.

10. A method of manufacturing the composition as defined in the claims 1-7 comprising furazidin and bulking agent, **characterized in that** furazidin in an amount from 0.5 to 95% by weight is mixed with a bulking agent chosen from the group consisting of starch, gelatinized starch, microcrystalline cellulose, lactose, glucose, mannitol, sorbitol, talc, anhydrous colloidal silica, dextrins or/and their mixture or/and additionally with a binding agent chosen from the group consisting of saccharides, polyhydric alcohols, polymers of acrylic acid derivatives, polymers of methacrylic acid derivatives, polymers of vinyl alcohol derivatives, chemically modified cellulose derivatives, polyvinylpyrrolidones and/or polyethylene oxides or/and an emulsifying agent chosen from the group consisting of phospholipids, polyoxyethylene sorbitan derivatives, fatty acids and/or fatty alcohols or/and a surfactant or/and a lubricant or/and a sweetening agent or/and a flavoring agent or/and a buffering agent.

## Patentansprüche

1. Pharmazeutische orale Zusammensetzung, die Furazidin enthält, **dadurch gekennzeichnet, dass** sie in Form eines Pulvers oder/und eines Granulats oder/und eines beschichteten Granulats vorliegt und von 0,5 Gew.-% bis 95 Gew.-% Furazidin und mindestens einen Füllstoff in einer Menge von 5 Gew.-% bis 99 Gew.-% enthält, der aus der Gruppe ausgewählt ist, die aus Stärke, gelatinierter Stärke, mikrokristalliner Cellulose, Lactose, Glucose, Mannit, Sorbit, wasserfreiem kolloidalem Siliciumdioxid, Talk, Dextrinen oder/und deren Gemisch besteht, und mindestens eine Verbindung mit Bindungs- und/oder Beschichtungseigenschaften, ausgewählt aus der Gruppe bestehend aus Sacchariden, mehrwertigen Alkoholen, Polymeren von Acrylsäurederivaten, Polymeren von Methacrylsäurederivaten, Polymeren von Vinylalkoholderivaten, chemisch modifizierten Cellulosederivaten, Polyvinylpyrrolidonen und/oder Polyethylenoxiden in einer Menge von 0,1 Gew.-% bis 30 Gew.-%.

2. Zusammensetzung nach Anspruch 1, **dadurch gekennzeichnet, dass** sie nicht modifizierte Freisetzungseigenschaften aufweist.

3. Zusammensetzung nach Anspruch 1, **dadurch gekennzeichnet, dass** sie modifizierte Freisetzungseigenschaften aufweist.

4. Zusammensetzung nach einem der vorhergehenden Ansprüche, **dadurch gekennzeichnet, dass** sie mindestens eine Verbindung mit emulgierenden Eigenschaften in einer Menge von 0,1 Gew.- % bis 45 Gew.-% enthält, die aus der Gruppe ausgewählt ist, die aus Phospholipiden, Polyoxyethylen-Sorbitan-Derivaten, Fettsäuren und/oder Fettalkoholen besteht.

5. Zusammensetzung nach einem der vorhergehenden Ansprüche, **dadurch gekennzeichnet, dass** sie mindestens eine Verbindung aus der Gruppe der Tenside in einer Menge von 0,1 Gew.-% bis 15 Gew.-% enthält.

6. Zusammensetzung nach einem der vorhergehenden Ansprüche, **dadurch gekennzeichnet, dass** sie mindestens ein Gleitmittel in einer Menge von 0,1 Gew.-% bis 10 Gew.-% enthält.

7. Zusammensetzung nach einem der vorhergehenden Ansprüche, **dadurch gekennzeichnet, dass** sie mindestens eine Substanz aus der Gruppe der Süßungsmittel, Aromastoffe und Puffermittel in einer Menge von 0,01 Gew.-% bis 25 Gew.-% enthält.

8. Verfahren zur Herstellung der in den Ansprüchen 1 bis 7 definierten Zusammensetzung, die Furazidin und einen Füllstoff enthält, **dadurch gekennzeichnet, dass** eine wässrige oder/und organische Lösung der Substanz mit Bindeeigenschaften ausgewählt aus der Gruppe bestehend aus Sacchariden, mehrwertigen Alkoholen, Polymeren von Acrylsäurederivaten, Polymeren von Methacrylsäurederivaten, Polymeren von Vinylalkoholderivaten, chemisch modifizierten Cellulosederivaten, Polyvinylpyrrolidonen und/oder Polyethylenoxiden mit einer Konzentration von 0,1 Gew.-% bis 30 Gew.-% hergestellt wird, dann Furazidin in einer Menge von 0,5o/o Gew.-% bis 95 Gew.-% mit der Substanz mit emulgierenden Eigenschaften und/oder mit dem Füllstoff gemischt wird, dann die Mischung von Furazidin mit der Substanz mit emulgierenden Eigenschaften und/oder mit dem Füllstoff unter Zugabe der Bindemittellösung granuliert wird, nach der Granulierung das erhaltene Granulat kalibriert und getrocknet wird und dann mit Füllstoff und/oder einem Gleitmittel und/oder einem Tensid und/oder einem Süßstoff und/oder einem Aromastoff und/oder einem Puffermittel gemischt wird.

9. Verfahren nach Anspruch 8, **dadurch gekennzeichnet, dass** das Granulat direkt nach dem Trocknen oder nach dem Trocknen und Kalibrieren und vor dem Mischen mit anderen Substanzen mit Überzugsmitteln beschichtet wird.

10. Verfahren zur Herstellung der in den Ansprüchen 1 bis 7 definierten Zusammensetzung, die Furazidin und Füllstoff enthält, **dadurch gekennzeichnet, dass** Furazidin in einer Menge von 0,5 bis 95 Gew.-% mit einem Füllstoff, ausgewählt aus der Gruppe, bestehend aus Stärke, gelatinierter Stärke, mikrokristalliner Cellulose, Lactose, Glucose, Mannit, Sorbit, Talk, wasserfreiem kolloidalem Siliciumdioxid, Dextrinen oder/und deren Gemisch oder/und zusätzlich mit einem Bindemittel, ausgewählt aus der Gruppe, bestehend aus Sacchariden, mehrwertigen Alkoholen, Polymeren von Acrylsäurederivaten, Polymeren von Methacrylsäurederivaten, Polymeren von Vinylalkoholderivaten, chemisch modifizierten Cellulosederivaten, Polyvinylpyrrolidonen und/oder Polyethylenoxiden oder/und einem Emulgator, ausgewählt aus der Gruppe bestehend aus Phospholipiden, Polyoxyethylensorbitanderivaten, Fettsäuren und/oder Fettalkoholen oder/und einem Tensid oder/und einem Gleitmittel oder/und einem Süßungsmittel oder/und einem Aromastoff oder/und einem Puffermittel.

## Revendications

1. Composition pharmaceutique orale comprenant de la furazidine, **caractérisée par le fait qu'**elle se présente sous forme de poudre et/ou de granulés et/ou de granulés enrobés, et qu'elle comprend de 0.5% en poids à 95% en poids de furazidine et qu'elle comprend au moins un agent de charge en une quantité allant de 5% en poids à 99% en poids, choisi dans le groupe constitué par l'amidon, l'amidon gélatinisé, la cellulose microcristalline, le lactose, le glucose, le mannitol, le sorbitol, la silice colloïdale anhydre, le talc, les dextrines ou/et leur mélange, et qu'elle comprend au moins un composé ayant des propriétés de liaison ou/et d'enrobage choisi dans le groupe constitué par les saccharides, les alcools polyhydriques, les polymères des dérivés de l'acide acrylique, les polymères des dérivés de l'acide méthacrylique, les polymères des dérivés de l'alcool vinylique, les dérivés de la cellulose modifiés chimiquement, les polyvinylpyrrolidones et/ou les oxydes de polyéthylène en une quantité allant de 0.1% en poids à 30% en poids.

2. Composition selon la revendication 1, **caractérisée par le fait qu'**elle présente des caractéristiques de libération non modifiées.

3. Composition selon la revendication 1, **caractérisée par le fait qu'**elle présente des caractéristiques de libération modifiées.

4. Composition selon l'une quelconque des revendications précédentes, **caractérisée par le fait qu'**elle comprend au moins un composé ayant des propriétés émulsifiantes en une quantité allant de 0.1% en poids à 45% en poids, choisi dans le groupe constitué par les phospholipides, les dérivés du polyoxyéthylène sorbitane, les acides gras et/ou les alcools gras.

5. Composition selon l'une quelconque des revendications précédentes, **caractérisée par le fait qu'**elle comprend au moins un composé choisi dans le groupe des tensioactifs en une quantité allant de 0.1% en poids à 15% en poids.

6. Composition selon l'une quelconque des revendications précédentes, **caractérisée en ce qu'**elle comprend au moins un lubrifiant en une quantité allant de 0.1% en poids à 10% en poids.

7. Composition selon l'une quelconque des revendications précédentes, **caractérisée en ce qu'**elle comprend au moins une substance choisie dans le groupe constitué des agents édulcorants, des agents aromatisants et des agents tampons en une quantité allant de 0.01% en poids à 25% en poids.

8. Procédé de fabrication de la composition telle que définie dans les revendications 1 à 7 comprenant de la furazidine et un agent de charge, **caractérisé par le fait qu'**on prépare une solution aqueuse ou/et organique de la substance ayant des propriétés de liaison choisie dans le groupe constitué par les saccharides, les alcools polyhydriques, les polymères des dérivés de l'acide acrylique, les polymères des dérivés de l'acide méthacrylique, les polymères des dérivés de l'alcool vinylique, les dérivés de la cellulose chimiquement modifiée, les polyvinylpyrrolidones et/ou les oxydes de polyéthylène ayant une concentration de 0.1% en poids à 30% en poids, puis la furazidine en une quantité allant de 0.5% en poids à 95% en poids est mélangée à la substance ayant des propriétés émulsifiantes et/ou à l'agent de charge, puis le mélange de furazidine avec la substance ayant des propriétés émulsifiantes et/ou avec l'agent de charge est granulée avec ajout de la solution de liant, après granulation les granulés obtenus sont calibrés et séchés, et ensuite ils sont mélangés à l'agent de charge et/ou à un lubrifiant et/ou à un surfactant et/ou à un agent édulcorant et/ou à un agent aromatisant et/ou à un agent tampon.

9. Procédé selon la revendication 8, **caractérisé par le fait que** les granulés sont enrobés avec des agents d'enrobage directement après le séchage ou après le séchage et le calibrage, et avant d'être mélangés à d'autres substances.

10. Procédé de fabrication de la composition telle que définie dans les revendications 1 à 7 comprenant de la furazidine et un agent de charge, **caractérisé par le fait que** la furazidine dans une quantité de 0.5 à 95% en poids est mélangée avec un agent de charge choisi dans le groupe constitué par l'amidon, l'amidon gélatinisé, la cellulose microcristalline, le lactose, le glucose, le mannitol, le sorbitol, le talc, la silice colloïdale anhydre, les dextrines ou/et leur mélange ou/et en outre avec un agent de liaison choisi dans le groupe constitué par les saccharides, les alcools polyhydriques, les polymères de dérivés de l'acide acrylique, les polymères de dérivés de l'acide méthacrylique, les polymères de dérivés de l'alcool vinylique, les polymères de dérivés de l'acide chlorhydrique, les polymères de dérivés de l'acide chlorhydrique, les polymères de dérivés de l'acide chlorhydrique, polymères de dérivés d'alcool vinylique, les dérivés de la cellulose chimiquement modifiés, les polyvinylpyrrolidones et/ou les oxydes de polyéthylène ou/et un agent émulsifiant choisi dans le groupe constitué par les phospholipides, les dérivés du polyoxyéthylène sorbitane, les acides gras et/ou les alcools gras ou/et un agent tensioactif ou/et un lubrifiant ou/et un agent édulcorant ou/et un agent aromatisant ou/et un agent tampon.
